(19) Europäisches Patentamt
European Patent Office
Office européen des brevets

(11) Numéro de publication : **0 162 757 B1**

# (12) FASCICULE DE BREVET EUROPÉEN

(45) Date de publication du fascicule du brevet : **24.01.90**

(21) Numéro de dépôt : **85400805.9**

(22) Date de dépôt : **24.04.85**

(51) Int. Cl.⁵ : **A 61 K 39/295**, A 61 K 39/285, A 61 K 39/205, C 12 N 15/00, A 61 K 39/42

(54) **Vaccin contre la rage et procédé pour sa préparation.**

(30) Priorité : **25.04.84 FR 8406499**

(43) Date de publication de la demande : **27.11.85 Bulletin 85/48**

(45) Mention de la délivrance du brevet : **24.01.90 Bulletin 90/04**

(84) Etats contractants désignés : **AT BE CH DE FR GB IT LI LU NL SE**

(56) Documents cités :
**EP-A- 0 083 286**
**EP-A- 0 140 762**
**NATURE, vol. 312, 8 novembre 1984, pages 163-166; M.P. KIENY et al.: "Expression of rabies virus glycoprotein from a recombinant vaccinia virus"**
**PROCEEDINGS OF THE NATIONAL ACADEMY OF SCIENCES USA, vol. 81, novembre 1984, pages 7194-7198; T.J. WIKTOR et al.: "Protection from rabies by a vaccinia virus recombinant containing the rabies virus glycoprotein gene"**
**Nature 302 p. 490-95 1983**
**Applied Virology p.234-40 1984**

(73) Titulaire : **TRANSGENE S.A.**
**16, rue Henri-Regnault**
**F-92400 Courbevoie (FR)**

(72) Inventeur : **Lathe, Richard**
**Arc Abro Kings Buildings**
**West Mains Road Edinbourgh EH9 (FR)**
Inventeur : **Kieny, Marie-Paule**
**11, rue de Gascogne**
**F-67000 Strasbourg (FR)**
Inventeur : **Drillien, Robert**
**10, Boulevard Paul Déroulède**
**F-67000 Strasbourg (FR)**
Inventeur : **Lecocq, Jean-Pierre**
**6, rue du Champ du Feu**
**F-67116 Reichsteet (FR)**

(74) Mandataire : **Warcoin, Jacques et al**
**Cabinet Régimbeau 26, avenue Kléber**
**F-75116 Paris (FR)**

**EP 0 162 757 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La rage est une maladie très ancienne mais, jusqu'à maintenant, son contrôle complet n'a pas pu être réalisé. Bien qu'il existe des vaccins efficaces contre la rage, de tels vaccins sont trop onéreux pour pouvoir être utilisés à titre préventif. En outre, il existe un réservoir très important de virus de la rage dans les animaux sauvages et pour cette raison, seuls des pays insulaires comme la Grande-Bretagne et le Japon, ont pû parvenir à une éradication de ce fléau.

L'agent causal de cette maladie est un Rhabdovirus. La transmission de la rage comporte en général la morsure d'un individu récepteur par un animal infecté ; le changement de comportement associé à l'infection chronique joue un rôle important dans l'étiologie de la maladie.

Dans l'homme, l'infection est suivie par une période de dormance pendant laquelle le virus voyage par le système nerveux jusqu'au cerveau. Au début, cette maladie peut être traitée de façon efficace par une vaccination intensive ; toutefois, lorsque les symptômes comportementaux apparaissent, la mort est presque inévitable.

Le virus comporte 5 protéines virales dont une seule, la glycoprotéine (G, haemmagglutinine), traverse la bicouche lipidique enveloppant le virus. Ainsi, la glycoprotéine est le seul composant viral capable de réagir avec des anticorps neutralisant le virus et également d'induire leur production.

Anilionis et al. (1981) ont décrit l'isolement d'une séquence codante correspondant à l'ARN messager de la glycoprotéine de la rage, souche ERA et Lathe et al. (1984) ont décrit l'expression de cette séquence dans une bactérie.

Des résultats similaires ont été rapportés par Yelverton et al. (1983) en utilisant une deuxième souche de rage, CVS.

Toutefois, une immunisation efficace contre le virus de la rage utilisant la matière synthétisée par les bactéries n'a pas encore pu être réalisée.

Des résultats préliminaires suggèrent que des modifications post-traductionnelles et/ou une présentation de la glycoprotéine sont des paramètres importants dans l'utilisation de cet antigène pour qu'il confère une protection contre le virus de la rage.

La présente invention décrit l'expression d'une séquence codant pour la glycoprotéine G de la rage dans un environnement tel qu'une modification et une présentation correctes des produits de traduction primaire puissent intervenir.

Deux groupes ont récemment mis en évidence l'utilisation de recombinants vivants du virus de la vaccine pour exprimer l'antigène de l'Influenza ou de l'Hépatite B pour immuniser contre ces maladies (Smith et al., 1983 ; Panicali et al., 1983).

L'expression d'une séquence codante pour une protéine exogène dans le virus de la vaccine (VV) implique nécessairement deux étapes :

1. La séquence codante doit être alignée avec un promoteur de VV et être insérée dans un segment non essentiel de l'ADN de VV cloné dans un plasmide bactérien approprié ;

2. Les séquences d'ADN de VV situées de part et d'autre doivent permettre des recombinaisons homologues in vivo entre le plasmide et le génome viral. Une double recombinaison réciproque conduit à un transfert de l'insert d'ADN du plasmide dans le génome viral dans lequel il est propagé et exprimé (Panicali et Paoletti, 1982 ; Mackett et al., 1982 ; Smith et al., 1983 ; Panicali et al., 1983).

La présente invention concerne un virus de la vaccine caractérisé en ce qu'il comporte tout ou partie d'une séquence d'ADN codant pour une glycoprotéine antigénique de la rage, nommée ci-après séquence d'ADN (I).

Par « virus de la vaccine », on entend désigner tout ou partie d'un virus du genre Poxvirus, en particulier du sous genre vaccine, qui incorpore, outre la vaccine elle-même, d'autres virus tels que le « Cowpox ».

On entend désigner par « glycoprotéine antigénique de la rage » une glycoprotéine qui, in vitro, mais de préférence in vivo, présente des caractéristiques immunogéniques identiques ou voisines de la glycoprotéine de la rage vraie, c'est-à-dire celle du virus sauvage.

Bien que l'on préfère utiliser pour la séquence ADN (I) la séquence d'ADN codant pour la protéine mature complète, il est possible d'utiliser seulement une partie de cette séquence d'ADN ou une telle séquence portant des mutations ponctuelles mais qui conduisent à des produits ayant des activités quasi identiques. Ce virus comportera de préférence l'ensemble des éléments assurant l'expression de ladite glycoprotéine, en particulier un promoteur d'un gène de la vaccine tel que le promoteur du gène de 7,5K noté P7,5K qui sera placé en amont de la séquence d'ADN (I).

Cet ensemble promoteur/séquence d'ADN (I) sera inséré dans un gène du virus de la vaccine, par exemple le gène TK, ce qui fournit une possibilité de sélection comme cela sera expliqué ci-après.

Le virus hybride ainsi obtenu peut être utilisé tel quel, vivant ou inactivé par traitement chimique ou physique, comme agent vaccinant ou bien être utilisé pour infecter une culture cellulaire de laquelle on pourra extraire la glycoprotéine antigénique par des techniques connues à partir du broyat cellulaire.

On peut encore envisager, pour réduire au maximum les risques d'accidents de vaccination avec un virus vivant, d'utiliser un mutant thermosensible de la vaccine (F. Keller et col., 1978 et F. Keller et R. Drillien, 1980.

En outre ce type de mutation ts peut exister sur le vecteur vaccine lui-même ou bien être introduit par mutation dans les virus recombinants selon l'invention. En particulier, on peut introduire dans le virus recombinant différentes thermosensibilités afin de maintenir le phénotype, même en cas de réversion partielle.

En outre, on peut prévoir l'utilisation d'un virus mutant de la vaccine présentant une spécificité d'hôte qui ne pousse pas ou peu sur des cellules humaines et dont la pathogénicité est encore plus faible que celle de la vaccine.

La présente invention concerne des vaccins destinés au traitement ou à la prévention de la rage, caractérisés en ce qu'il est constitué d'un virus de la vaccine recombinant comportant une séquence d'ADN codant pour la glycoprotéine G du virus de la rage.

Pour ces vaccins, les voies d'administration peuvent être variées, en particulier on peut procéder par voie intradermique ou orale. Ces vaccins peuvent être administrés avec des supports pharmaceutiques connus et comporter, en outre, des adjuvants permettant d'augmenter leur pouvoir vaccinant.

Les exemples ci-après sont destinés à mettre en évidence d'autres caractéristiques et avantages de la présente invention.

La préparation du virus selon l'invention comporte notamment les étapes suivantes :

1) une restructuration d'une extrémité du cADN de la rage, schématisée à la figure 1, par le procédé schématisé à la figure 2, pour obtenir pTG155 ;

2) une mutation d'une des séquences de ce cADN afin de rétablir dans la glycoprotéine la proline au 8ème acide aminé, figure 3, pour obtenir ptg155-pro ;

3) la synthèse d'un mini-plasmide ptg1H à partir de pBR322 (figure 4) ;

4) l'insertion dans ce mini-plasmide du fragment Hin-J portant le gène TK de VV ;

5) l'insertion dans le gène TK du promoteur de la protéine 7,5K (figure 5) ;

6) l'insertion sous le promoteur de P7,5K du gène de la rage porté par ptg155-pro (figure 5) ;

7) le clonage des éléments essentiels de ce dernier plasmide dans le virus de la vaccine (figure 6).

Les exemples suivants illustrent les propriétés des différents composants obtenus.

Les différents matériels mis en œuvre sont identifiés dans les exemples.

Sauf indication contraire, les enzymes sont utilisées dans les conditions préconisées par le fabricant et les techniques mises en œuvre sont connues de l'homme de métier.

Les séquences d'acides aminés et les séquences de nucléotides représentées sur les figures ne sont pas reprises dans le corps de la description pour ne pas l'alourdir mais elles en constituent une partie intégrante.

Restructuration du cADN de la glycoprotéine de la rage

La séquence codant pour la glycoprotéine de la rage publiée par Anilionis et al. et représentée à la figure 1, comporte des éléments qui peuvent gêner sa traduction et son expression comme antigène.

Le cADN de la glycoprotéine de la rage qui provient du plasmide pRG décrit par Anilionis et col. (1981) et est porté, sur un fragment BglII/BglII, par différents plasmides comme pTG147, pTG171, pTG150, a déjà été décrit dans le brevet français n° 83 15716, (WO-A-85/1516).

Tout d'abord, l'ATG correspondant au codon d'initiation pour le message mature se trouve immédiatement adjacent à une séquence polyG introduite par la procédure de clonage du cADN. Cette séquence est susceptible de provoquer une instabilité lors de la recombinaison in vivo entre les éléments G/C à chacune des extrémités du cADN et peut de plus gêner la traduction du message.

Ensuite, les séquences du cADN de la glycoprotéine ne correspondent pas parfaitement à la séquence codant pour la partie N-terminale de la glycoprotéine mature. C'est pourquoi il a été décidé d'éliminer ces deux obstacles avant d'effectuer le clonage de la séquence codante correspondant.

Kozak (1981, 1983) a montré que les codons d'initiation de la traduction dans les eucaryotes se trouvent normalement dans une séquence qui peut être représentée de la façon suivante : A ** ATG (A ou G) et des altérations de cette séquence peuvent conduire à des diminutions de la traduction. Bien que l'importance de ces nucléotides d'extrémité ne soit pas parfaitement établie, il semble préférable d'éliminer la séquence G/C adjacente et de conserver l'essence de la séquence consensus mentionnée précédemment. En outre, il est nécessaire de prévoir une séquence de reconnaissance BglII en amont de façon à faciliter la manipulation subséquente.

En restructurant l'extrémité 5' du cADN, comme cela est représenté à la figure 2, on a profité de l'existence d'un site unique MstII qui recouvre les codons codant pour les amino-acides 2, 3 et 4 du produit de traduction primaire.

Dans le plasmide pTG150, le cADN de la glycoprotéine de la rage d'Anilionis a été inséré, en utilisant des oligo-nucléotides adaptateurs PstI/BglII (Lathe et al. 1982) dans un site unique BglII inséré dans le site HindIII du plasmide pBR327. (Soberon et col., 1980).

Ce plasmide, représenté en A de la figure 2, a été mis en digestion partielle avec BglII et en digestion complète avec MstII et les fragments linéaires appropriés ont été isolés par électrophorèse sur un gel d'agarose à basse température de gélification. Les oligonucléotides (5'-dGATCTAATATGGTTCC-3') et (5'-dTGAGGAACCATATTA-3') ont été synthétisés selon une technique précédemment décrite (Kohli et al., 1982). Les oligonucléotides sont hybridés pour donner un duplex partiel (B de la figure 2) et insérés entre

les extrémités BglII et MstII du plasmide linéaire purifié, ce qui permet la recircularisation et génère le plasmide pTG155 (C de la figure 2).

Le 8ème amino-acide de la glycoprotéine mature (ne présentant pas la séquence signal) des souches de virus ERA et CVS de la rage est la proline, tandis que dans le cADN isolé par Anilionis et al., on trouve, à cette position, un triplet codant pour la leucine.

Dans pTG155 représenté à la figure 3, le site mutant est flanqué par un site en amont EcoRI dans le vecteur et par un site HindIII sur les séquences correspondant aux amino-acides 10 et 11 de la séquence codant pour la glycoprotéine mature. Ce petit fragment EcoRI/HindIII est cloné dans le bactériophage M13tg131 (Kieny et al., 1983) en orientation inverse. Une mutagénèse localisée dirigée par un oligonucléotide est effectuée selon la technique de Zoller et Smith (1983) en utilisant l'oligonucléotide (5'-dATCACGATCCCAGACAAGC-3') synthétisé comme cela a été indiqué précédemment.

Le codon pour l'amino-acide 8 dans la séquence de cADN est changé pour remplacer CTA en CCA.

L'oligonucléotide (19-mer) contient deux modifications par rapport à la séquence originale : un T en place de C, ce qui corrige le triplet de la leucine CTA en proline CCA, et un A en place de C qui correspond à une transversion dans la séquence GATA. Ceci génère une séquence GATC de dam méthylation ce qui favorise l'incorporation de la séquence d'oligonucléotide par correction des faux appariments sélectifs in vivo.

Il faut remarquer que le fragment EcoRI/HindIII qui a été cloné en orientation inverse de celle qui est présentée ici de façon que le brin inférieur, complémentaire de l'oligonucléotide, soit présent dans le bactériophage recombinant M13 mono-brin.

Des plages de phages sont examinées pour l'hybridation dans des conditions appropriées avec l'oligonucléotide marqué à son extrémité 5' par $^{32}P$. Un certain nombre de plages positives sont prélevées et les inserts correspondants sont séquencés. L'insert de l'un des clones correctement modifié est recloné dans pTG155 en échangeant les fragments EcoRI/HindIII pour générer finalement le plasmide restructuré pTG155-pro. L'analyse des clones apparaissant pendant cette étape est facilitée par l'existence d'un nouveau site pour l'enzyme de restriction Sau3A qui coïncide avec la séquence de méthylation dam introduite pendant la mutagénèse dirigée par oligonucléotide.

Dans le plasmide pTG155-pro, le cADN de la glycoprotéine de la rage restructuré est flanqué à chacune de ses extrémités par des sites BglII. A l'extrémité aval le site BglII provient de l'utilisation de l'oligonucléotide adaptateur BglII/PstI octamer utilisé pour cloner le fragment de cADN PstI/PstI dans le site BglII et le site en amont résulte de l'utilisation d'un petit oligonucléotide double brin destiné à éliminer l'extrémité G/C.

Construction des plasmides hybrides

Les tailles combinées des différents éléments nécessaires pour le transfert de la séquence codant pour la glycoprotéine de la rage dans le génome de VV et son expression subséquente sont de l'ordre de plusieurs kb. Il a donc été jugé nécessaire de minimiser la taille du plasmide de réplication dans E. coli utilisé pour le travail de construction de façon à faciliter les manipulations nécessaires.

Le fragment HindIII (Hin-J) du génome de VV contient le gène complet de la thymidine kinase (TK) qui a déjà été utilisé précédemment pour permettre l'échange et la recombinaison de l'ADN inséré dans le génome de VV (Mackett et al., 1982). Il est important de noter que le transfert d'un insert dans le gène TK dans le génome de VV crée un virus TK déficient qui peut être reconnu par simple sélection.

Il a tout d'abord été nécessaire de produire un plasmide de petite taille portant un site unique HindIII utilisable pour l'intégration du fragment Hin-J VV. En outre, il était nécessaire d'éliminer les séquences de restriction non nécessaire du plasmide de façon à permettre les manipulations suivantes.

La construction a été amorcée à partir du plasmide pML2 (Lusky et Botchan, 1981) qui est un vecteur dérivé du plasmide pBR322 par délétion spontanée dans lequel le segment entre le nucléotide 1089 et 2491 a été perdu (figure 4). D'abord la séquence de PstI a été éliminée par insertion du fragment AhaIII-AhaIII de pUC8 (Vieira et Messing, 1982) entre deux sites AhaIII de pML2 en éliminant 19 paires de bases. On a utilisé la méthode de « linker-tailing » (Lathe et al., 1984) pour insérer un linker HindIII entre les sites NruI et EcoRI traité par SI de ce plasmide, en éliminant le site BamHI. Ceci conduit à un plasmide de 2049 paires de bases portant le gène β-lactamase fonctionnel (conférant la résistante à l'ampicilline et comportant en outre une origine de réplication active dans E. coli et un site de restriction unique HindIII.

Cette construction a été appelée pTG1H.

Le fragment Hin-J de l'ADN de VV portant le gène TK a préalablement été cloné dans un vecteur provenant de pBR327 (Drillien et Spehner, 1983). Ce fragment de 4,6 kb a été recloné dans le site HindIII de pTG1H. Un clone a été sélectionné dans lequel le gène TK est situé distalement par rapport au gène codant pour la résistance à l'ampicilline.

Cette construction pTG1H-TK a été utilisée comme porteur dans l'expérience suivante.

L'étape suivante a été d'isoler un promoteur de VV utilisable pour commander l'expression de la séquence codant pour la glycoprotéine de la rage insérée. Le promoteur d'un gène précoce codant pour une protéine de 7 500 daltons (7,5K) a déjà été utilisé avec succès dans un but identique (Smith et al., 1983) et on a donc procédé à l'isolement de ce segment.

Le gène 7,5K est situé sur l'un des plus petits fragments SalI (fragment Sal-S) du génome de VV type WR (Venkatasan et al., 1981). Comme les petits fragments sont clonés de façon préférentielle, une grande proportion des clones obtenus par clonage direct de l'ADN de VV type WR coupé par SalI dans le plasmide pBR322 porte le fragment Sal-S. Ce fragment est transféré sur le bactériophage vecteur M13mp701 (voir Kieny et al., 1983), par digestion SalI et religation, en conduisant ainsi au phage M13tgSal-S.

Dans ce clone, un site ScaI se trouve immédiatement à proximité de l'ATG d'initiation du gène 7,5K. En aval du gène 7,5K se trouvent situés des sites uniques BamHI et EcoRI provenant du vecteur. Les sites BamHI et ScaI sont fusionnés par l'intermédiaire d'un linker BglII 5'-CAGATCTG-3' par la technique des « linker » après avoir complété les extrémités générées par digestion BamHI avec le fragment Klenow de la polymérase. Ce procédé élimine le site ScaI mais reconstitue le site BamHI et déplace le site unique EcoRI en aval. En même temps, le site SalI (AccI) en aval est éliminé, le site en amont devient donc unique.

Cette construction est appelée M13tg7,5K.

A l'intérieur du fragment Hind-J de l'ADN de VV se trouvent situés des sites ClaI et EcoRI qui sont séparés par environ 30 paires de bases (Weir et Moss, 1983). Le fragment pomoteur de 7,5K présent dans M13tg7,5K est excisé par AccI et EcoRI et cloné entre les sites ClaI et EcoRI de pTG1H-TK pour générer pTG1H-TK-P7,5K dont la synthèse est schématisée figure 5.

Cette construction conduit au transfert du site BamHI unique du vecteur M13 immédiatement en aval de la séquence du promoteur 7,5K. Ce site unique BamHI est utilisé dans les constructions suivantes.

pTG1H-TK-P7,5K est mis en digestion avec BamHI et ligué avec pTG155-pro digéré par BglII. (Figure 5).

Après transformation de E. coli, l'un des plasmides recombinants isolé par cette procédure, pVVgRAB, est sélectionné car il porte le cADN de la glycoprotéine de la rage dans l'orientation correcte pour l'expression à partir du promoteur de 7,5K.

pVVgRAB sera parfois noté pTG1H-TK-P7,5K-gRAB.

Clonage dans le virus de la vaccine (figure 6)

La stratégie décrite par Smith et al. (1983) repose sur l'échange in vivo entre un plasmide portant un insert dans le gène VV TK et le génome viral de type sauvage de façon à inactiver le gène TK porté par le virus. Les virus TK$^-$ peuvent être sélectionnés par étalement sur une lignée cellulaire TK-négative en présence de 5-bromodéoxyuridine (5BUdR) (Mackett et al., 1982). La thymidine kinase phosphoryle le5BUDR en 5'-monophosphate, qui est ensuite converti en triphosphate. Ce composé est un analogue de dTTP et son incorporation dans l'ADN bloque le développement correct du virus. Un virus TK$^-$ peut néanmoins répliquer son ADN normalement et il conduit à des plaques virales visibles dans une couche cellulaire également TK$^-$.

Le virus de la vaccine se propage dans le cytoplasme des cellules infectées plutôt que dans leur noyau. C'est pourquoi il n'est pas possible de tirer avantage de la machinerie de réplication et de transcription de l'ADN de l'hôte et il est nécessaire que le virion possède les composants pour l'expression du gène viral. L'ADN de VV purifié est non-infectieux.

Afin de générer les recombinants, il est nécessaire d'effectuer simultanément l'infection cellulaire avec un VV et une transfection avec le segment d'ADN cloné qui présente de l'intérêt. Toutefois, la génération des recombinants est limitée à une petite proportion des cellules compétentes pour la transfection à l'ADN. C'est pour cette raison qu'il a été nécessaire de mettre en œuvre une stratégie de « congruence » indirecte pour réduire le bruit de fond des virus parentaux non-recombinants. Ceci a été effectué en utilisant comme virus infectieux vivant un mutant thermosensible (ts) de la vaccine qui n'est pas capable de se propager à une température non permissive de 39,5 °C (Drillien et Spehner, 1983). Lorsque les cellules sont infectées avec un mutant ts dans des conditions non permissives et transfectées avec l'ADN d'un virus de type sauvage, la multiplication virale interviendra seulement dans les cellules qui sont compétentes pour la transfection et dans lesquelles une recombinaison entre l'ADN viral sauvage et le génome du virus ts aura eu lieu ; aucun résultat ne sera obtenu dans les autres cellules, en dépit du fait qu'elles ont été infectées. Si un plasmide recombinant contenant un fragment de l'ADN de vaccine tel que pVVgRAB est inclus dans le mélange de transfection à la concentration appropriée avec l'ADN du type sauvage, il est également possible d'obtenir qu'il participe à la recombinaison homologue avec l'ADN de la vaccine dans les cellules compétentes.

Des monocouches de cellules primaires de fibroblastes d'embryons de poulets (CEF) sont infectées à 33 °C avec VV-copenhague ts 26 (0,1 pfu/cellule) et transfectées avec un coprécipité au phosphate de calcium de l'ADN de type sauvage VV-Copenhague (0,5 $\mu g/10^6$ cellules) et de plasmide recombinant pTG1H-TK-P7,5-gRAB (3,0 $\mu g/10^6$ cellules). Il faut remarquer que les concentrations plus faibles (0,1 $\mu g/10^6$ cellules) ont dans les expériences suivantes donné des rendements sensiblement améliorés en recombinants.

Après incubation pendant 2 heures à une température qui ne permet pas le développement du virus ts (39,5 °C), les cellules sont rincées et incubées pendant 48 heures à 39,5 °C. Des dilutions de virus ts$^+$ sont utilisées pour réinfecter une monocouche de cellules de souris L-TK$^-$ à 37 °C et incubées en présence de 5BUdR (100 $\mu g/ml$). Différentes plaques TK$^-$ sont obtenues à partir de ces cellules qui ont reçu le plasmide recombinant, tandis que les cultures de contrôle sans plasmide ne montrent pas de plaques visibles.

Une double recombinaison réciproque correcte entre le plasmide hybride rage/vaccine et le génome de VV échange le gène viral TK pour le gène TK porté par l'insert présent dans le plasmide. Dans le génome de VV le gène TK est présent sur un fragment unique HindIII :Hin-J. Les recombinants ayant transféré le bloc d'expression de la glycoprotéine de la rage sont sensés avoir intégré un site interne HindIII dérivant du cADN de la glycoprotéine. C'est pourquoi les ADN purifiés à partir de virus TK⁻ sont digérés avec HindIII et soumis à une électrophorèse sur gel d'agarose. Comme cela était prévisible, le fragment Hin-J de 4,6 kb est absent, par contre deux nouveaux fragments de 1,1 et 5,5 kb révèlent la présence d'un insert contenant un site interne HindIII. Après sous clonage des virus TK⁻ l'un des recombinants VVgRAB-26D3 est conservé.

Expression de la glycoprotéine de la rage à partir des virus de vaccine recombinants

Des mono-couches de cellules L-TK⁻ semi-confluentes sont infectées avec VVgRAB-26D3 (50 pfu/cellule) pendant 1 heure à la température ambiante puis un milieu de culture exempt de méthionine est ajouté, et après 30 mm chaque boîte de culture est complémentée avec de la L-méthionine $35_{SI}$ (1 265 Ci/mmole) et l'incubation est poursuivie à 37 °C pendant 4 heures. Les cellules sont récoltées, resuspendues dans un tampon d'immunoprécipitation contenant des inhibiteurs de protéase et après éclatement aux ultra-sons et clarification, les protéines fixées par l'anti-sérum anti-rage sont récupérées par chromatographie d'affinité sur une résine protéine A-sépharose et chargées sur un gel d'électrophorèse et fluorographiées par une technique décrite par Lathe et al., (1980).

Un anti-sérum polyclonal 3554-R215 dressé contre la glycoprotéine purifiée au Wistar Institute a été utilisé dans les expériences illustrées dans la figure 7, A (a) et B ; des résultats identiques sont obtenus (b, c) avec deux anticorps monoclonaux neutralisant les virus (509-6 et 101-1 du Wistar) qui identifient différents épitopes sur la glycoprotéine de la rage. En A la monocouche cellulaire est infectée avec :

1) le recombinant VVgRAB-26D3,
2) le virus de la vaccine du type sauvage,
3) aucun virus.

En (B) une mono-couche cellulaire est infectée avec VVgRAB-26D3 et marquée en présence (1) ou absence (2) du tunicamycine (2 μ/ml).

Dans (A) et (B) les poids moléculaires standards sont donnés en kilodaltons.

Comme cela apparaît dans la figure 7A, un anti-sérum rabique polyclonal de même que deux anticorps monoclonaux neutralisent le virus, précipitent une protéine qui migre sous forme d'une bande diffuse avec un poids moléculaire apparent d'environ 66 000 daltons. Ce poids moléculaire correspond précisément au poids, 67 000 daltons, d'une glycoprotéine rabique authentique.

Comme le poids moléculaire de la glycoprotéine naturelle et du recombinant correspondent, on a vérifié que la glycoprotéine recombinante était glycosylée in vivo. Une mono-couche cellulaire a été infectée avec VVgRAB-2603 comme précédemment, et on a ajouté au milieu avant le marquage un inhibiteur puissant de la glycosylation, la tunicamycine (2 μg/ml). Les extraits de cellules infectées sont immunoprécipités en utilisant un anticorps polyclonal et le produit est chargé sur un gel d'électrophorèse comme précédemment. Comme le montre la figure 7B, l'addition de tunicamycine au milieu conduit à une réduction du poids moléculaire de la glycoprotéine recombinante de 66 000 daltons, ceci correspond à l'élimination des groupements glycosylés de la protéine. On peut donc en conclure que la glycoprotéine rabique recombinante est glycosylée dans les cellules infectées par VVgRAB. Différentes bandes additionnelles dans la ligne (1) sont présumées être des produits de dégradation de l'antigène de la rage non glycosylé.

Dans une autre expérience, une mono-couche de cellules L-TK⁻ est infectée avec VVgRAB-26D3 ($10^5$ pfu par plaque contenant $10^6$ cellules) et incubée pendant 8 heures à 37 °C. Les cellules sont fixées, traitées avec des anticorps monoclonaux et polyclonaux et après un lavage très complet, l'anticorps fixé est détecté en utilisant un deuxième anticorps (chèvre anti-souris) marqué par fluoresceine. La plupart des cellules infectées par le recombinant VVg-RAB-26D3 (A) montrent une fluorescence significative tandis que les cellules non infectées (C) et les cellules infectées avec les virus non-recombinants (B) ne montrent pas de fluorescence.

Comme cela ressort de la figure 8, la fluorescence est associée en priorité avec la membrane cytoplasmique comme cela pouvait être attendu d'une protéine transmembranaire.

Propriétés immunologiques in vivo du virus hybride vaccine/rage vivant

Des lapins sont immunisés par intra-dermo avec $3 \times 10^7$ pfu de VVgRAB-26D3 et des échantillons de sérum sont prélevés au bout de 0, 11 et 14 jours. Une enflure reconnaissable est observée au site d'infection qui diminue après 8 à 9 jours. Une souche de virus rabique ERA inactivée avec de la β-propiolactone et marquée avec $I^{125}$ selon le protocole standard et testée pour sa réaction avec le sérum des animaux immunisés.

Les protéines fixées sont placées sur gel d'électrophorèse et autoradiographiées.

Les sérums de 11 et 14 jours, mais pas le sérum du jour de contrôle 0, se sont trouvés reconnaître et fixer efficacement la glycoprotéine virale radiomarquée. Le sérum des animaux de contrôle immunisés avec le virus de la vaccine non-recombinant de type Copenhague ne donne pas de telles réactions. Le sérum des animaux immunisés décrit précédemment, est ensuite testé pour l'inactivation du virus rabique in vitro. Les dilutions de la souche ERA rabique sont préincubées pendant 1 heure avec différentes quantités de l'anti-sérum de lapin et étalés sur des cellules de reins de hamsters nouveau-nés (BHK) sur des plaques à microtitration ($10^3$ cellules/puits). Après incubation à 37 °C pendant 22 heures, les cellules infectées productrices sont colorées en utilisant une technique d'immunofluorescence directe.

Le tableau I indique que même pour les dilutions les plus élevées de l'anti-sérum de 11 et 14 jours une inactivation complète du virus est obtenue. Les sérums préimmuns et non recombinants ne donnent pas de neutralisation détectable. Les titres sont donnés comme la plus forte dilution à laquelle on observe l'inhibition de l'infection.

Tableau I

| Nbre de jours après la vaccination | VVgRAB26D3 | | | | Vaccine Type Sauvage |
|---|---|---|---|---|---|
| | Lapin I | Lapin II | Lapin III | Lapin IV | |
| 0 | 0 | 0 | 0 | 0 | 0 |
| 11 | 10000 | 10000 | 10000 | 10000 | 0 |
| 14 | >30000 | >30000 | >30000 | >30000 | 0 |

Ces résultats démontrent que le recombinant VVgRAB-26D3 n'induit pas seulement la production d'anticorps réagissant avec le virus rabique, mais également que l'anti-sérum d'animaux immunisés est capable d'inactiver le virus rabique in vitro. L'induction d'anticorps neutralisant n'est pas toujours en corrélation avec une protection contre le développement de la maladie. C'est pourquoi il a été effectué une étude directe d'un test de protection en utilisant le virus recombinant. Des souris sont immunisées par injection dans la patte ou grattage sur la queue avec $10^7$ pfu de VVgRAB-26D3 vivant. Ces animaux sont ensuite inoculés avec une dose létale (1 000 unités, DL50) d'un virus rabique sauvage introduit par injection intra-cérébrale. Après 10 jours, le groupe des animaux de contrôle immunisés avec la vaccine non recombinante présente une infection rabique terminale, au contraire, 15 des 15 animaux immunisés avec VVgRAB-26D3 ne montrent aucune trace de maladie.

On peut donc conclure que l'immunisation avec le virus recombinant vaccine/rage vivant conduit à une protection contre la rage.

Propriétés immunologiques in vivo du virus hybride vaccine/rage inactivé

Trois types de vaccin inactivé peuvent être préparés à partir des cellules infectées par le virus recombinant vaccine/rage : soit un extrait brut de cellules infectées, soit du virus intact purifié, soit la glycoprotéine G purifiée. Les trois préparations sont inactivées au β-propiolactone.

Des cellules BHK infectées par VVgRAB-26D3 sont homogénéisées au broyeur de Dounce, et le surnageant de centrifugation représente l'extrait brut de cellules.

Pour obtenir le virus purifié tué, cette préparation est inactivée au β-propiolactone 1/4000 et centrifugée sur un gradient de sucrose selon des techniques classiques.

La glycoprotéine G purifiée est obtenue par solubilisation de l'extrait brut en présence de Triton X-100 2 %. Après centrifugation 1 heure à 100 000 g, G est isolée à partir du surnageant grâce à un passage sur une colonne d'affinité préparée avec un anticorps monoclonal anti-G. Cette glycoprotéine G purifiée est également inactivée au β-propiolactone.

Des souris sont immunisées par 2 injections intra-péritonéales de 0,5 ml de ces préparations inactivées à 1 semaine d'intervalle, et soumises à l'épreuve 1 semaine plus tard (240 unités DL50) par voie intracérébrale.

Le tableau II indique les anticorps anti-rabiques mesurés aux jours 7 et 14.

(Voir tableau II page 8)

Tableau II

| | Quantité de protéines injectées en µg/souris | Titre des anticorps anti-rabiques | |
|---|---|---|---|
| | | Jour 7 | Jour 14 |
| VVgRAB-26D3 extrait brut | 140 | 80 | 8 000 |
| VVgRAB-26D3 virus purifié | 9 | 270 | 4 000 |
| VVgRAB-26D3 G purifiée | 50 | 120 | 15 000 |
| Vaccine type Copenhagen extrait brut | 900 | 10 | 10 |

Tous les animaux vaccinés avec les différentes préparations de vaccin inactivé dérivé de VVgRAB-26D3 survivent à l'injection intracérébrale de virus rabique, alors que les animaux du groupe contrôle meurent dans les délais attendus.

Il est important de noter que, même inactivé, le virus hybride VVgRAB-26D3 confère une protection efficace contre l'infection rabique expérimentale. Ceci démontre que dans le virus recombinant intact, la glycoprotéine de la rage est présentée à la surface du virion et est susceptible d'induire une réponse immunologique semblable à celle induite par le virus de la rage inactivé.

Vaccination des renards et vaccination par voie orale

En Europe de l'Ouest, le renard est le principal agent de dissémination de la rage. Il est donc fondamental de pouvoir maîtriser la vaccination du renard. De préférence, celle-ci se fera par voie orale afin de minimiser la manipulation des animaux.

Les renards roux (Vulpes vulpes), âgés de moins d'un an, sont immunisés par diverses voies avec $10^8$ pfu de VVgRAB-26D3 vivant. Les témoins sont composés de 2 renards vaccinés avec du vaccin antirabique classique tué, et de 4 renards vaccinés avec la vaccine type sauvage.

Le tableau III donne le titre d'anticorps anti-rabiques mesurés au jour 7, 14 et 28. Ce tableau montre que chez le renard comme chez la souris, le virus recombinant vaccine-rage induit une production d'anticorps comparable à celle induite par le vaccin classique.

Les animaux vaccinés sont soumis à l'épreuve de l'injection de virus rabique virulent, au jour 28.

Les renards vaccinés avec le virus type sauvage meurent dans les délais attendus. Tous les renards vaccinés avec le vaccin tué classique ou avec VVgRAB-26D3 (même le renard (2) inoculé par voie sous-cutanée et qui ne présentait pas d'anticorps séro-neutralisants) sont vivants 2 mois après l'épreuve.

(Voir tableau page 9)

| Vaccin utilisé | Voie d'inoculation | Titre d'anticorps * | | |
|---|---|---|---|---|
| | | jour 6 | jour 14 | jour 18 |
| Classique tué | sous cutané (1)<br>(2) | 0,33<br>0,07 | 0,64<br>0,05 | 0,24<br>0,07 |
| Vaccine type sauvage | intra-dermique | - | - | - |
| VVgRAB-26D3 | intra-dermique (1)<br>(2) | 0,64<br>0,05 | 6,1<br>0,6 | 4,4<br>1,67 |
| VVgRAB-26D3 | sous-cutané (1)<br>(2) | 0,46<br>- | 2,32<br>- | 4,3<br>- |
| VVgRAB-26D3 | orale avec scarification des muqueuses (1)<br>(2) | 0,33<br>0,24 | 0,88<br>0,88 | 1,67<br>2,31 |

* Les titres en anticorps sont exprimés en unités internationales (sérum de référence — 65 U.I. — neutralisant à $10^{-4,2}$).

La souche suivante a été déposée dans la Collection Nationale de Cultures de Microorganismes (CNCM) — 28, rue du Docteur Roux 75724 PARIS CEDEX 15 :

E. Coli TGE1106 transformée par pTG171 no. I-248 déposée le 30 septembre 1983.

## References


Anilionis, A., Wunner, W. H. & Curtis, p. J. (1981) Nature 294, 275-278.

Drillien, R. & Spehner, D. (1983) Virology 131, 385-393.

Kieny, M. P., Lathe, R. & Lecocq, J. P. (1983) Gene 26, 91-99.

Kohli, V., Balland, A., Wintzerith, M., Sauerwald, R., Staub, A. & Lecoq, J. P. (1982) Nucleic Acids Res. 10, 7439-7448.

Kozak, M. (1981) Nucleic Acids Res. 9, 5233-5252.

Kozak, M. (1983) Microbiol. Rev. 47, 1-45.

Lathe, R., Hirth, P., Dewilde, M., Harford, N. & Lecocq, J. P. (1980) Nature 284, 473-474.

Lathe, R., Balland, A., Kohli, V. & Lecocq, J. P. (1982) Gene 20, 187-195.

Lathe, R., Kieny, M. P., Schmitt, D., Curtis, P. & Lecocq, J. P. (1984a) J. Mol. Appl. Genet., in press.

Lathe, R., Kieny, M. P., Skory, S. & Lecocq, J. P. (1984a) DNA, in press.

Lusky, M., Botchan, M. (1981) Nature 293, 79-81.

Mackett, M., Smith, J. L. & Moss, B. (1982) Proc. Natl. Acad. Sci. USA 79, 7415-7419.

Panicali, D. & Paoletti, E. (1982), Proc. Natl. Acad. Sci. USA 79, 4927-4931. Panicali, D., Davis, S. W., Weinberg, R. L. & Paoletti, E. (1983) Proc. Natl. Acad. Sci. USA 80, 5364-5368.

Smith, G. L., Mackett, M. & Moss, V. (1983) Nature 302, 490-495.

Soberon et coll., Gene 9, 287-305 (1980).

Venkatesan, S., Baroudy, B. M. & Moss, B. (1981) Cell 125, 805-813.

Vieira, J. & Messing, J. (1982) Gene 19, 259-268.

Weir, J. P. & Moss, B. (1983) J. Virol. 46, 530-537.

Wiktor, T. J. (1978) Develop. Biol. Standard 40, 255-264.

Wiktor, T. J. (1980) in Rhabdoviruses III pp. 99-112 (ed. D. H. L. Bishop) CRC Press, Inc.

Wunner, W. H., Dietzschold, B., Curtis, P. J. & Wiktor, T. J. (1983) J. Gen. Virol. 64, 1649-1659.

Yelverton, E., Norton, S., Obijeski, J. F. & Doeddel, D. V ; (1983) Science 219, 614-620.

Zoller, M. J. & Smith, M. (1983) in Methods in Enzymology, Vol. 100, pp. 468-500 (Eds. Wu, R., Grossman, L., Moldave, K ;) Academic Press, London.

Keller F., Drillien R. et Kirn A. : Thermosensibilité du développement des poxvirus et virulence. Utilisation de souches thermosensibles comme vaccin. Rencontre Biologique, 1978 (L. Hartmann), p. 121-126, Ed. Varia, Paris.

Keller F. et Drillien R. : Un mutant thermosensible atténué du virus vaccinal. Ann. Virol. (Institut Pasteur), 1980, 131 E, 85-94.

## Revendications

1. Vaccin destiné au traitement et à la prévention de la rage, caractérisé en ce qu'il est constitué d'un virus de la vaccine recombinant comportant une séquence d'ADN codant pour la glycoprotéine G du virus de la rage.

2. Vaccin selon la revendication 1, caractérisé en ce que la séquence d'ADN codant pour la glycoprotéine G de la rage est la séquence d'ADN codant pour la glycoprotéine mature complète.

3. Vaccin selon la revendication 1, caractérisé en ce que la séquence d'ADN codant pour la glycoprotéine G de la rage est sous le contrôle d'un promoteur de la vaccine.

4) Vaccin selon la revendication 3, caractérisé en ce que le promoteur de la vaccine est le promoteur du gène de la protéine 7,5 K de la vaccine.

5. Vaccin selon l'une des revendications 1 à 4, caractérisé en ce que la séquence codant pour la glycoprotéine G de la rage est insérée dans un gène non essentiel de la vaccine.

6. Vaccin selon la revendication 5, caractérisé en ce que la séquence codant pour la glycoprotéine G de la rage est clonée dans le gène TK de la vaccine.

7. Vaccin selon l'une des revendications 1 à 6, caractérisé en ce que le virus de la vaccine est thermosensible.

8. Vaccin selon l'une des revendications 1 à 7, caractérisé en ce que le virus est vivant.

9. Vaccin selon l'une des revendications 1 à 7, caractérisé en ce que le virus est inactivé.

## Claims

1. A vaccine for treatment and prevention of rabies, characterised in that it is formed by a virus of the recombinant vaccine comprising a DNA sequence which codes for the G glycoprotein of the rabies virus.

2. A vaccine according to claim 1, characterised in that the DNA sequence which codes for the G glycoprotein of rabies is the DNA sequence which codes for the complete mature glycoprotein.

3. A vaccine according to claim 1, characterised in that the DNA sequence which codes for the G glycoprotein of rabies is under the control of a promotor of the vaccine.

4. A vaccine according to claim 3, characterised in that the promotor of the vaccine is the promotor of the gene of the 7,5 K protein of the vaccine.

5. A vaccine according to any of claims 1 to 4, characterised in that the sequence which codes for the rabies G glycoprotein is inserted into a non-essential gene of the vaccine.

6. A vaccine according to claim 5, characterised in that the sequence which codes for the rabies G glycoprotein is cloned in the TK gene of the vaccine.

7. A vaccine according to any of claims 1 to 6, characterised in that the vaccine virus is heat-sensitive.

8. A vacine according to any of claims 1 to 7, characterised in that the virus is alive.

9. A vaccine according to any of claims 1 to 7, characterised in that the virus is inactivated.

## Patentansprüche

1. Vakzine für die Behandlung und Prävention der Rabies, dadurch gekennzeichnet, daß sie besteht aus einem Virus der Rekombinanten-Vakzine, die eine ADN-Sequenz trägt, die für das Glycoprotein G des Rabies-Virus kodiert.

2. Vakzine nach Anspruch 1, dadurch gekennzeichnet, daß die ADN-Sequenz, die für das Glycoprotein G der Rabies kodiert, die ADN-Sequenz ist, die für das komplette reife Glycoprotein kodiert.

3. Vakzine nach Anspruch 1, dadurch gekennzeichnet, daß die ADN-Sequenz, die für das Glycoprotein G der Rabies kodiert, unter der Kontrolle eines Vakzine-Promotors steht.

4. Vakzine nach Anspruch 3, dadurch gekennzeichnet, daß es sich bei dem Vakzine-Promotor um den Promotor des Gens des Proteins 7,5 K der Vakzine handelt.

5. Vakzine nach einem der Ansprüche 1 bis 4, dadurch gekennzeichnet, daß die Sequenz, die für das Glycoprotein G der Rabies kodiert, in ein nicht-essentielles Gen der Vakzine eingesetzt ist.

6. Vakzine nach Anspruch 5, dadurch gekennzeichnet, daß die Sequenz, die für das Glycoprotein G der Rabies kodiert, in dem Gen TK der Vakzine geklont ist.

7. Vakzine nach einem der Ansprüche 1 bis 6, dadurch gekennzeichnet, daß das Virus der Vakzine wärmeempfindlich ist.

8. Vakzine nach einem der Ansprüche 1 bis 7, dadurch gekennzeichnet, daß das Virus lebend ist.

9. Vakzine nach einem der Ansprüche 1 bis 7, dadurch gekennzeichnet, daß das Virus inaktiviert ist.

ORABPST.DAT MOD.

PstI -19 1
1 CTG CAG GGG GGG GGG GGG GGA GGA AAG ATG GTT CCT CAG GCT CTC CTG TTT GTA CCC 61 CTT CTG GTT TTT CCA TTG TGT TTT GGG AAA TTC
Met Val Pro Gln Ala Leu Leu Phe Val Pro Leu Leu Val Phe Pro Leu Cys Phe Gly Lys Phe

HindIII HgiJII PvuII
91 CCT ATT TAC ACG ATA CTA GAC AAG CTT 121 GGT CCC TGG AGC CCG ATT GAC ATA CAT CAC CTC AGC TGC CCA AAC AAT TTG GTA GTG GAG GAC
Pro Ile Tyr Thr Ile Leu Asp Lys Leu Gly Pro Trp Ser Pro Ile Asp Ile His His Leu Ser Cys Pro Asn Asn Leu Val Val Glu Asp

181 GAA GGA TGC ACC AAC CTG TCA GGG TTC 211 TCC TAC ATG GAA CTT AAA GTT GGA TAC ATC 241 TTA GCC ATA AAA ATG AAC GGG TTC ACT TGC ACA
Glu Gly Cys Thr Asn Leu Ser Gly Phe Ser Tyr Met Glu Leu Lys Val Gly Tyr Ile Leu Ala Ile Lys Met Asn Gly Phe Thr Cys Thr

271 GGC GTT GTG ACG GAG GCT GAA ACC TAC 301 ACT AAC TTC GTT GGT TAT GTC ACA ACC ACG 331 TTC AAA AGA AAG CAT TTC CGC CCA ACA CCA GAT
Gly Val Val Thr Glu Ala Glu Thr Tyr Thr Asn Phe Val Gly Tyr Val Thr Thr Thr Phe Lys Arg Lys His Phe Arg Pro Thr Pro Asp

AvaIII HpaII BstEII
GCA TGT AGA GCC GCG TAC AAC TGG AAG ATG GCC GGT GAC CCC AGA TAT GAA GAG TCT 421 CTA CAC AAT CCG TAC CCT GAC TAC CGC TGG CTT
Ala Cys Arg Ala Ala Tyr Asn Trp Lys Met Ala Gly Asp Pro Arg Tyr Glu Glu Ser Leu His Asn Pro Tyr Pro Asp Tyr Arg Trp Leu

AsuII XhoI AvaI
CGA ACT GTA AAA ACC ACC AAG GAG TCT 481 CTC GTT ATC ATA TCT CCA AGT GTA GCA GAT 511 TTG GAC CCA TAT GAC AGA TCC CTT CAC TCG AGG
Arg Thr Val Lys Thr Thr Lys Glu Ser Leu Val Ile Ile Ser Pro Ser Val Ala Asp Leu Asp Pro Tyr Asp Arg Ser Leu His Ser Arg

AvaI
541 GTC TTC CCT AGC GGG AAG TGC TCA GGA 571 GTA GCG GTG TCT TCT ACC TAC TGC TCC ACT 601 AAC CAC GAT TAC ACC ATT TGG ATG CCC GAG AAT
Val Phe Pro Ser Gly Lys Cys Ser Gly Val Ala Val Ser Ser Thr Tyr Cys Ser Thr Asn His Asp Tyr Thr Ile Trp Met Pro Glu Asn

631 CCG AGA CTA GGG ATG TCT TGT GAC ATT 661 TTT ACC AAT AGT AGA GGG AAG AGA GCA TCC 691 AAA GGG AGT GAG ACT TGC GGC TTT GTA GAT GAA
Pro Arg Leu Gly Met Ser Cys Asp Ile Phe Thr Asn Ser Arg Gly Lys Arg Ala Ser Lys Gly Ser Glu Thr Cys Gly Phe Val Asp Glu

StuI HaeI AhaIII SphI NruI
721 AGA GGC CTA TAT AAG TCT TTA AAA GGA GCA TGC AAA CTC AAG TTA TGT GGA GTT CTA 781 GGA CTT AGA CTT ATG GAT GGA ACA TGG GTC GCG
Arg Gly Leu Tyr Lys Ser Leu Lys Gly Ala Cys Lys Leu Lys Leu Cys Gly Val Leu Gly Leu Arg Leu Met Asp Gly Thr Trp Val Ala

FIG.1a

```
811
ATG CAA ACA TCA AAT GAA ACC AAA TGG TGC CCT CCC GAT CAG TTG GTG AAC CTG CAC GAC TTT CGC TCA GAC GAA ATT GAG CAC CTT GTT
Met Gln Thr Ser Asn Glu Thr Lys Trp Cys Pro Pro Asp Gln Leu Val Asn Leu His Asp Phe Arg Ser Asp Glu Ile Glu His Leu Val
                                                                  BgaI                    AcyI
901
GTA GAG GAG TTG GTC AGG AAG AGA GAG GAG TGT CTG GAT GCA CTA GAG TCC ATC ATG ACA ACC AAG TCA GTG AGT TTC AGA CGT CTC AGT
Val Glu Glu Leu Val Arg Lys Arg Glu Glu Cys Leu Asp Ala Leu Glu Ser Ile Met Thr Thr Lys Ser Val Ser Phe Arg Arg Leu Ser

991
CAT TTA AGA AAA CTT GTC CCT GGG TTT GGA AAA GCA TAT ACC ATA TTC AAC AAG ACC TTG ATG GAA GCC GAT GCT CAC TAC AAG TCA GTC
His Leu Arg Lys Leu Val Pro Gly Phe Gly Lys Ala Tyr Thr Ile Phe Asn Lys Thr Leu Met Glu Ala Asp Ala His Tyr Lys Ser Val

1081
AGA ACT TGG AAT GAG ATC CTC CCT TCA AAA GGG TGT TTA AGA GTT GGG GGG AGG TGT CAT CCT CAT GTG AAC GGG GTG TTT TTC AAT GGT
Arg Thr Trp Asn Glu Ile Leu Pro Ser Lys Gly Cys Leu Arg Val Gly Gly Arg Cys His Pro His Val Asn Gly Val Phe Phe Asn Gly

1171
ATA ATA TTA GGA CCT GAC GGC AAT GTC TTA ATC CCA GAG ATG CAA TCA TCC CTC CTC CAG CAA CAT ATG GAG TTG TTG GAA TCC TCG GTT
Ile Ile Leu Gly Pro Asp Gly Asn Val Leu Ile Pro Glu Met Gln Ser Ser Leu Leu Gln Gln His Met Glu Leu Leu Glu Ser Ser Val
                                  AccI
1261
ATC CCC CTT GTG CAC CCC CTG GCA GAC CCG TCT ACC GTT TTC AAG GAC GGT GAC GAG GCT GAG GAT TTT GTT GAA GTT CAC CTT CCC GAT
Ile Pro Leu Val His Pro Leu Ala Asp Pro Ser Thr Val Phe Lys Asp Gly Asp Glu Ala Glu Asp Phe Val Glu Val His Leu Pro Asp
                          HindII                                                      ApaI HglJII
1351
GTG CAC AAT CAG GTC TCA GGA GTT GAC TTG GGT CTC CCG AAC TGG GGG AAG TAT GTA TTA CTG AGT GCA GGG GCC CTG ACT GCC TTG ATG
Val His Asn Gln Val Ser Gly Val Asp Leu Gly Leu Pro Asn Trp Gly Lys Tyr Val Leu Leu Ser Ala Gly Ala Leu Thr Ala Leu Met
                                              ClaI°
1441
TTG ATA ATT TTC CTG ATG ACA TGT TGT AGA AGA GTC AAT CGA TCA GAA CCT ACG CAA CAC AAT CTC AGA GGG ACA GGG AGG GAG GTG TCA
Leu Ile Ile Phe Leu Met Thr Cys Cys Arg Arg Val Asn Arg Ser Glu Pro Thr Gln His Asn Leu Arg Gly Thr Gly Arg Glu Val Ser
                                                                                      505
1531
GTC ACT CCC CAA AGC GGG AAG ATC ATA TCT TCA TGG GAA TCA CAC AAG AGT GGG GGT GAG ACC AGA CTG TGA GGA CTG GCC GTC CTT TCA
Val Thr Pro Gln Ser Gly Lys Ile Ile Ser Ser Trp Glu Ser His Lys Ser Gly Gly Glu Thr Arg Leu ***
                                                                                                                 PstI
1621
ACG ATC CAA GTC CTG AAG ATC ACC TCC CCT TGG GGG GTT CTT TTT AAA AAA AAA AAA AAA AAA AAA AAA ACC CCC CCC CCC CCC CTG CAG
                                                                                                                 1710
```

FIG_1b

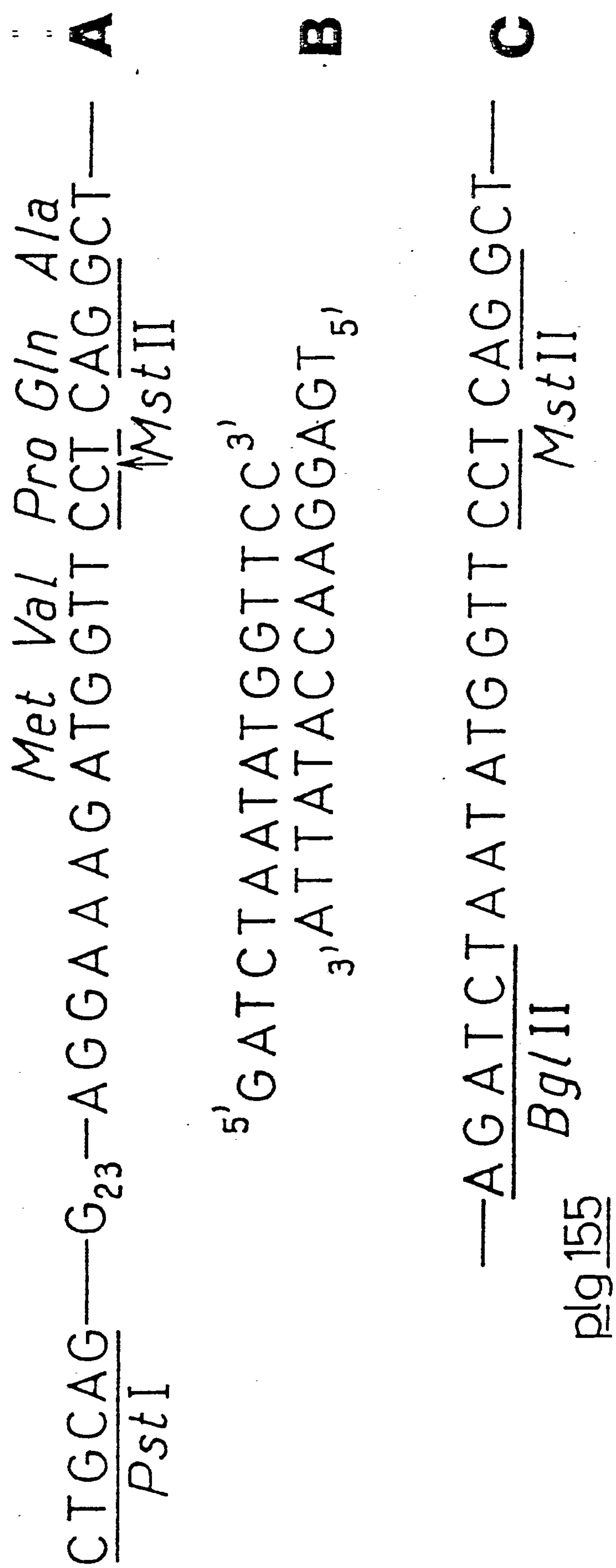
Met Val Pro Gln Ala
CTGCAG—G23—AGGAAAAGATGGTTCCTCAGGCT—
PstI
MstII
A
5'GATCTAATATGGTTCC3'
3'ATTATACCAAGGAGT5'
B
—AGATCTAATATGGTTCCTCAGGCT—
BglII
MstII
pIg155
C

FIG. 2

HindIII
EcoRI BglII
Met --------------- TyrThrIleLEUAspLysLeu ----
GAATTC---AGATCT---ATG--------------TACACGATACTAGACAAGCTT----

* *
5'-TACACGATCCCAGACAAGC-3'
****

HindIII
EcoRI BglII
Met --------------- TyrThrIlePROAspLysLeu ----
GAATTC---AGATCT---ATG--------------TACACGATCCCAGACAAGCTT----

FIG_3

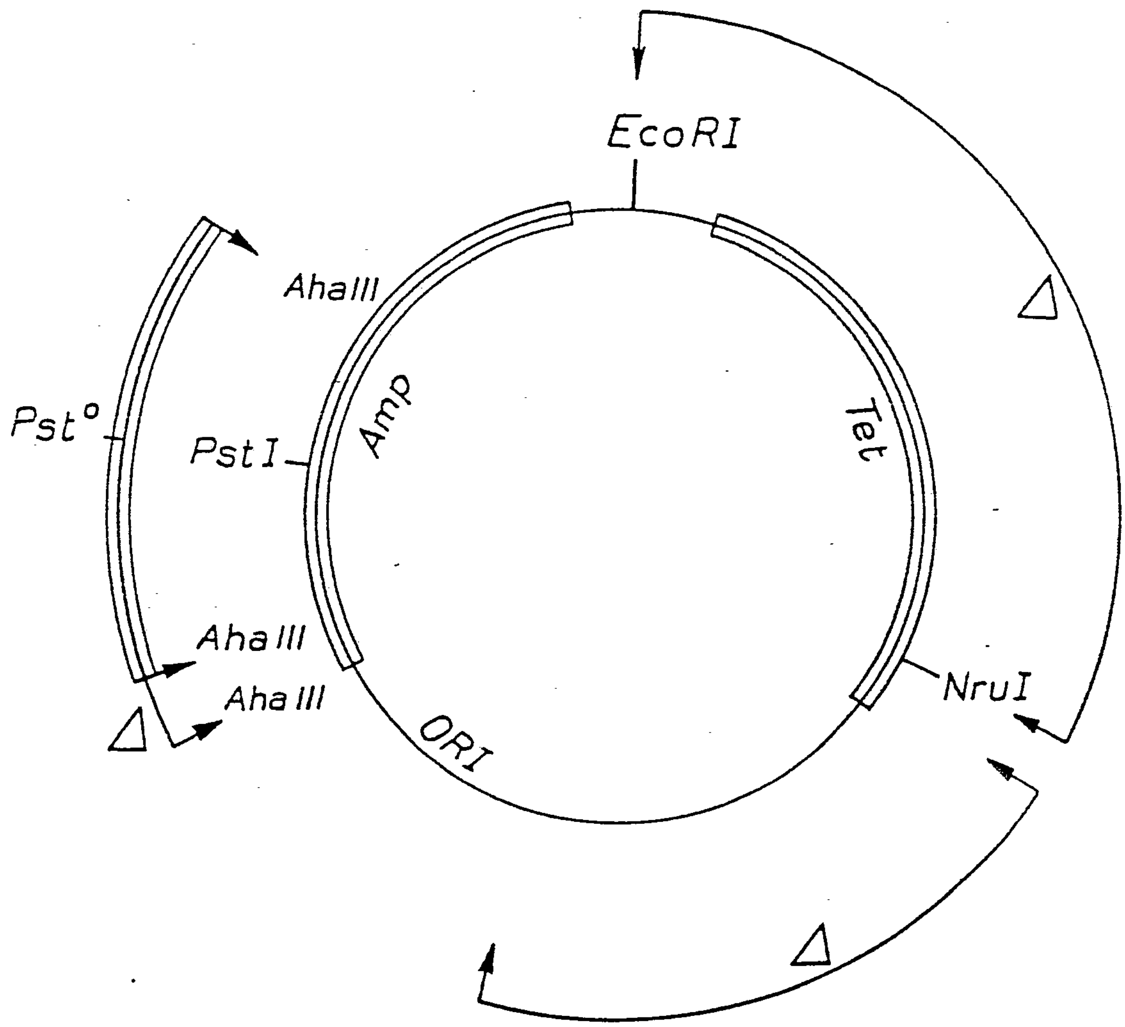


FIG_4

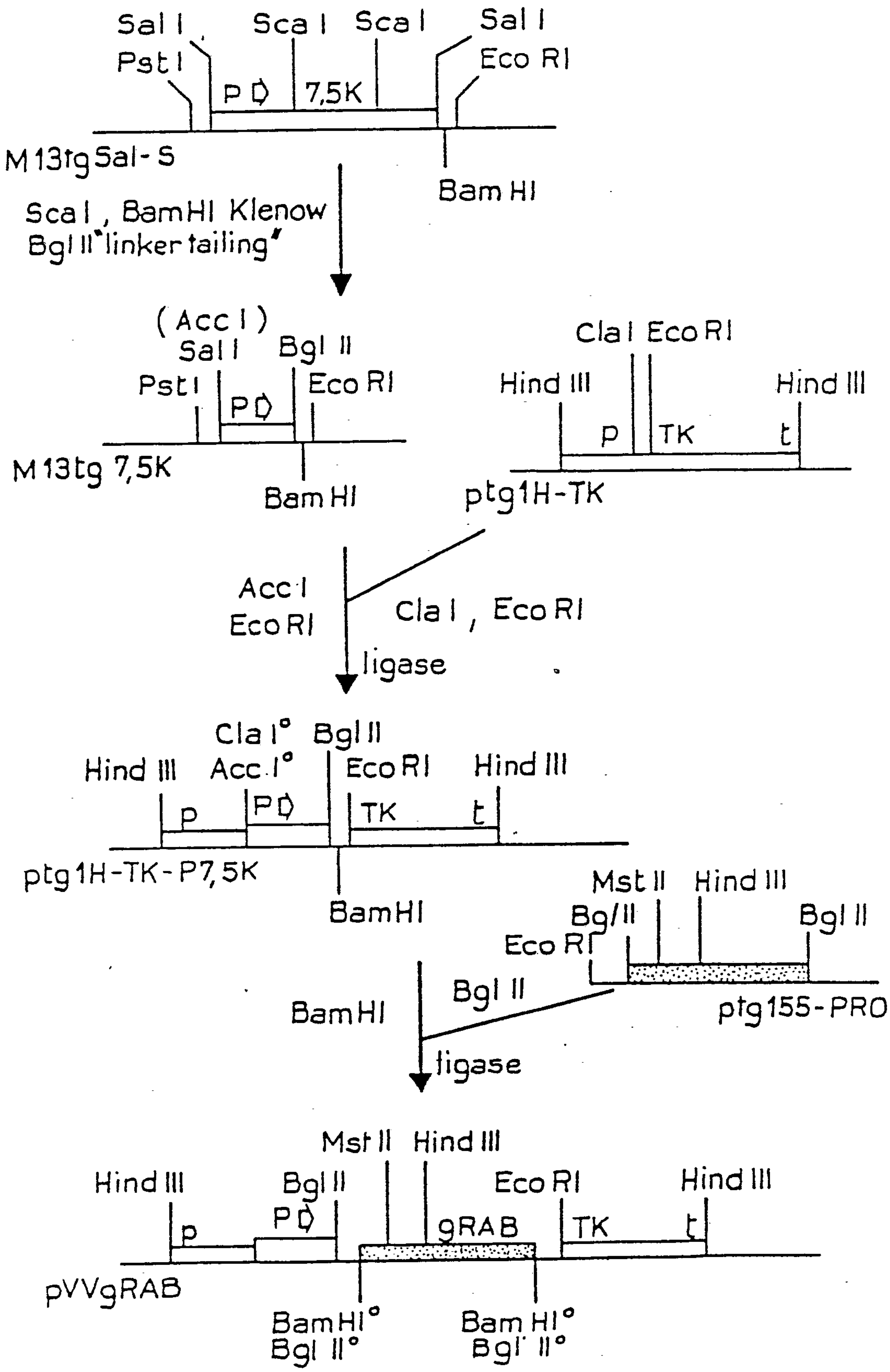
Sal I
Sca I
Sca I
Sal I
Pst I
Eco RI
P
7,5K
M13tg Sal-S
Bam HI
Sca I, BamHI Klenow
BglII "linker tailing"
(Acc I)
Sal I
Bgl II
Pst I
Eco RI
P
M13tg 7,5K
Bam HI
Cla I
Eco RI
Hind III
Hind III
p
TK
t
ptg1H-TK
Acc I
Eco RI
Cla I, Eco RI
ligase
Cla I°
Bgl II
Hind III
Acc I°
Eco RI
Hind III
p
P
TK
t
ptg1H-TK-P7,5K
BamHI
Mst II
Hind III
Bg/II
Bgl II
Eco RI
BamHI
Bgl II
ptg155-PRO
ligase
Mst II
Hind III
Hind III
Bgl II
Eco RI
Hind III
p
P
gRAB
TK
t
pVVgRAB
BamHI°
Bgl II°
Bam HI°
Bgl II°

FIG_5

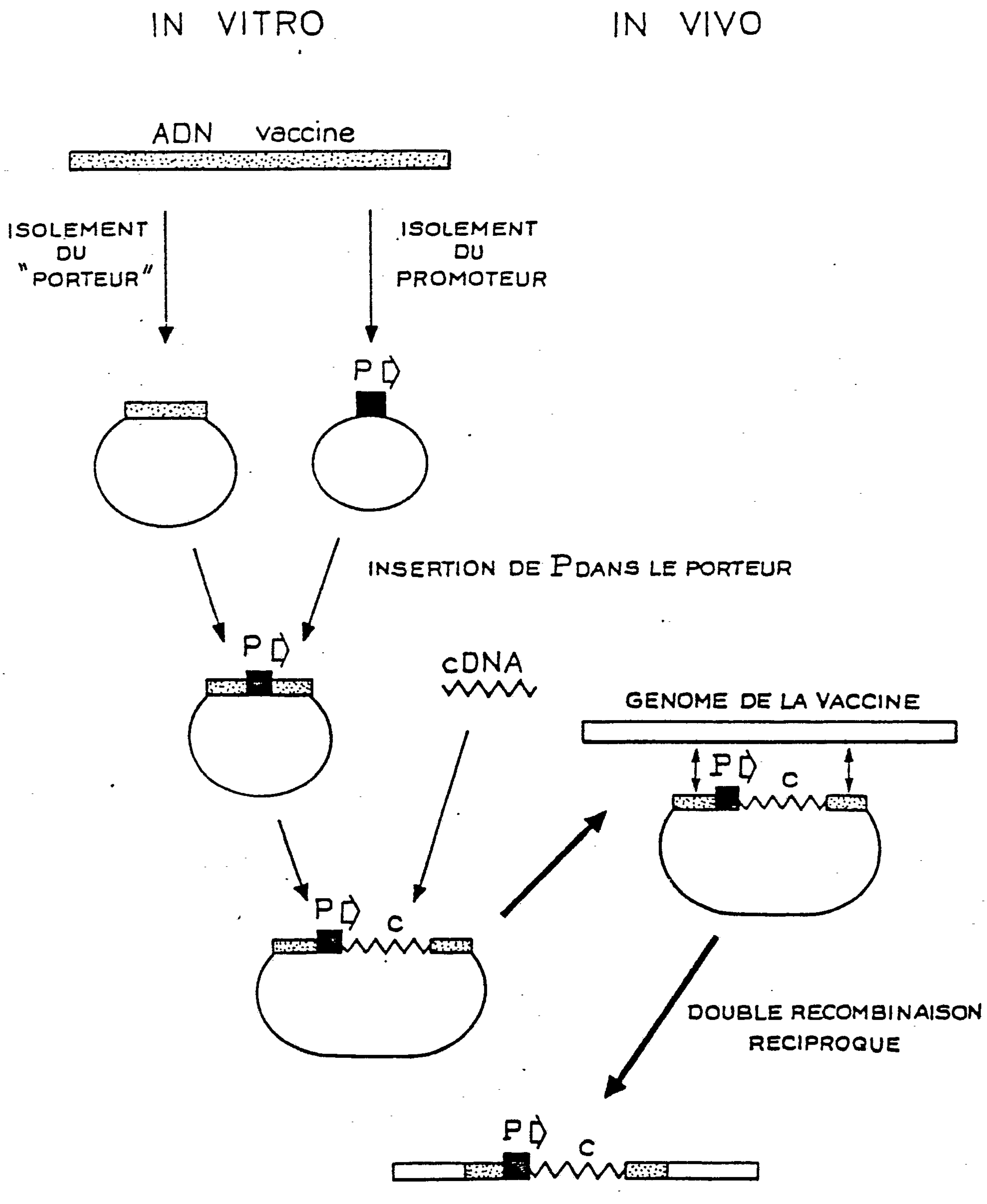
IN VITRO
IN VIVO
ADN vaccine
ISOLEMENT DU "PORTEUR"
ISOLEMENT DU PROMOTEUR
P
INSERTION DE P DANS LE PORTEUR
P
cDNA
GENOME DE LA VACCINE
P
c
P
c
DOUBLE RECOMBINAISON RECIPROQUE
P
c

FIG_6

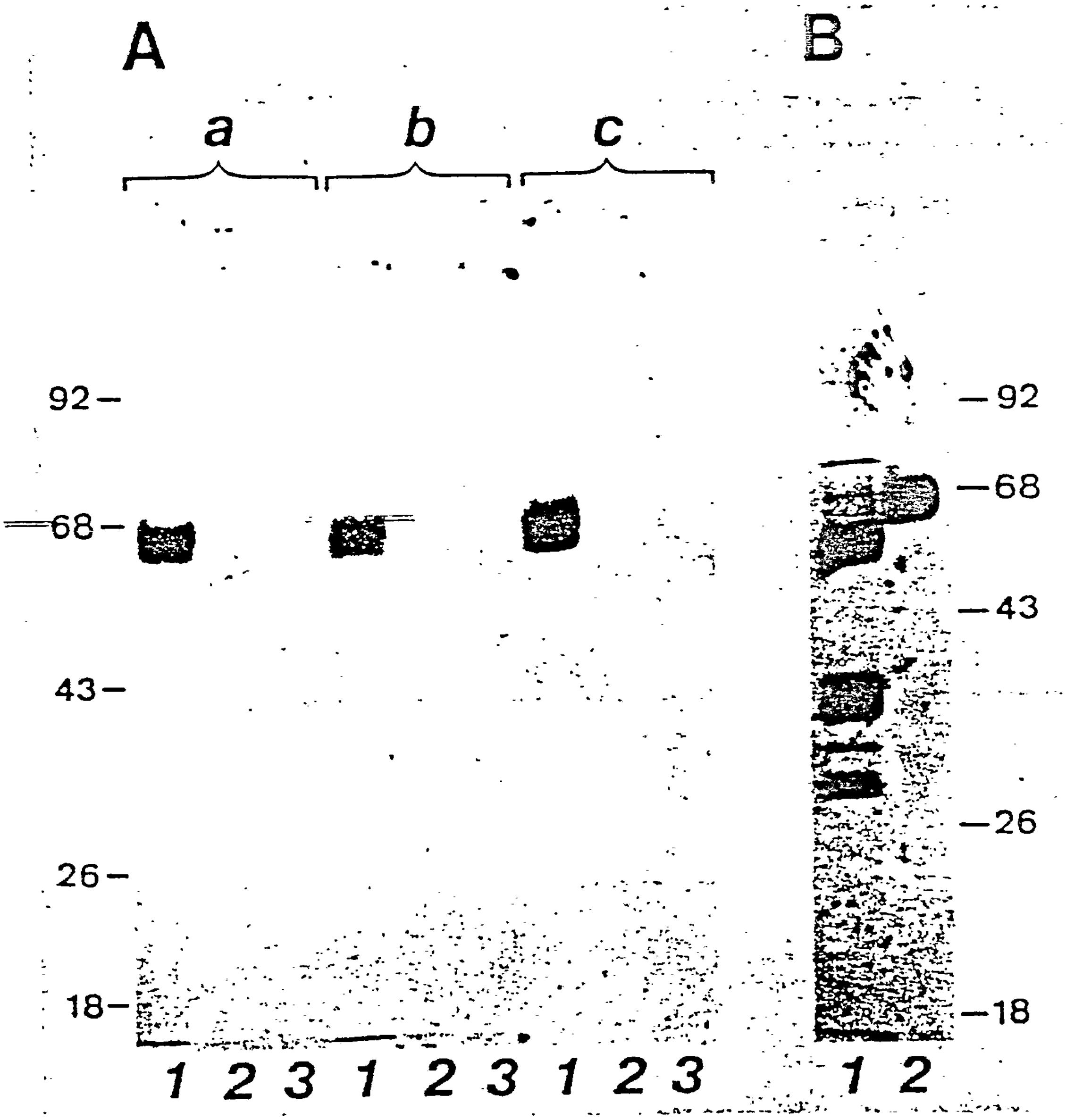


FIG_7

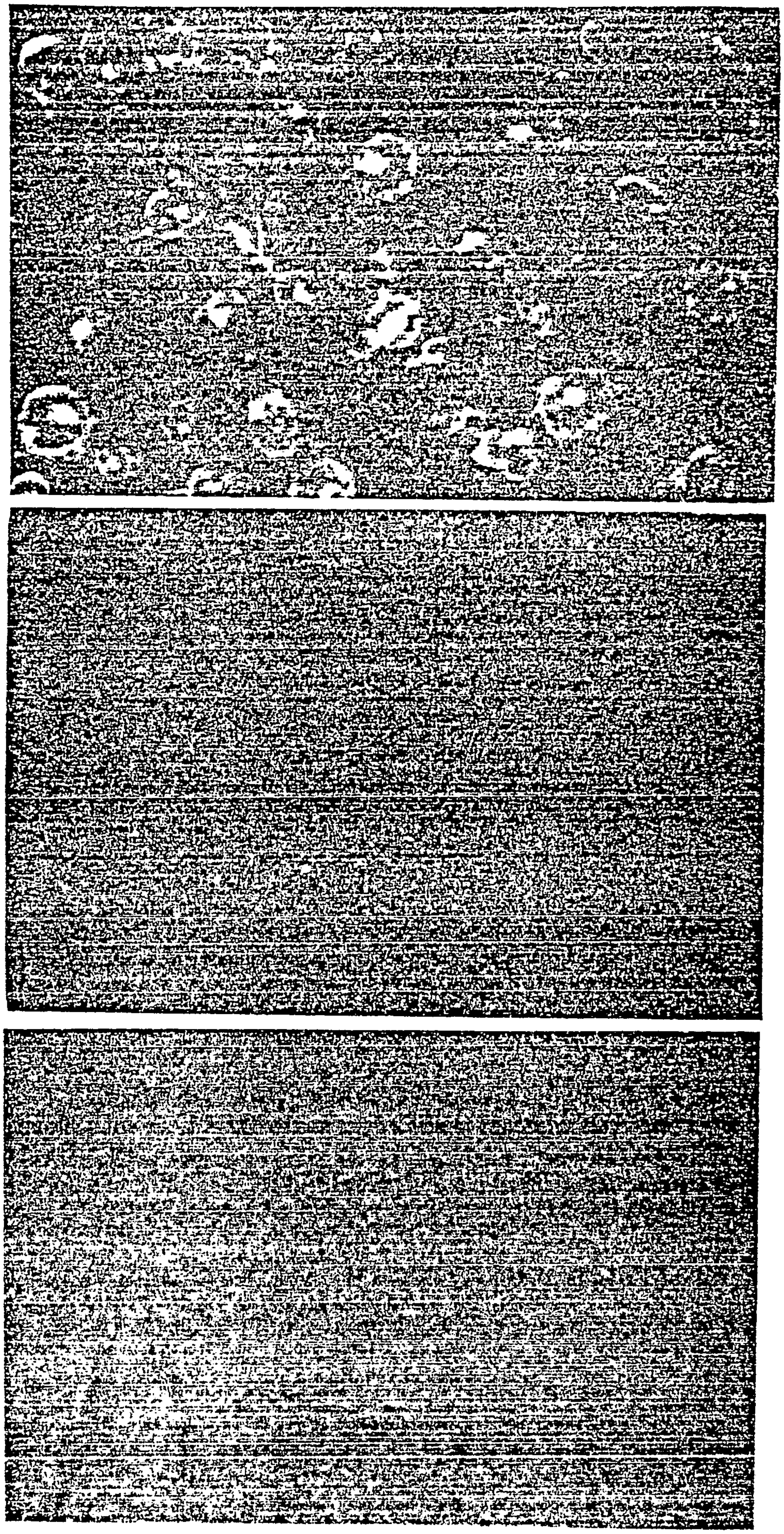


FIG_8